# EUROPEAN PATENT SPECIFICATION

(11) **EP 3 276 346 B1**
(45) Date of publication and mention of the grant of the patent: **06.01.2021**
(21) Application number: 17182680.3
(22) Date of filing: 21.07.2017
(51) Int. Cl.: G01N 33/28, F16N 29/04

(54) **MONITORING DEBRIS IN A LUBRICANT CONDUIT OF A TURBINE ENGINE**
ÜBERWACHUNG VON ABRIEB IN DER SCHMIERÖLLEITUNG EINES TURBINENMOTORS
SUPERVISION DE DÉBRIS DANS UNE LIGNE DE GRAISSAGE D'UN MOTEUR À TURBINE

(30) Priority: 25.07.2016 US 201615218307
(43) Date of publication of application: 31.01.2018
(73) Proprietor: United Technologies Corporation, Farmington, CT 06032 (US)
(72) Inventor: Hagen, Gregory S., Glastonbury, CT 06033 (US); Lin, Yiqing, Glastonbury, CT 06033 (US); Khibnik, Alexander I., Glastonbury, CT 06033 (US); Harris, Meggan, Colchester, CT 06415 (US); Erdinc, Ozgur, Coventry, CT 06238 (US); Giering, Michael J., Bolton, CT 06043 (US)
(74) Representative: Dehns

(56) References cited:
- EP-A1- 2 530 367
- EP-A2- 2 014 877
- WO-A1-2015/122224

## Description

### BACKGROUND

A gas turbine engine typically includes a fan section, a compressor section, a combustor section and a turbine section. Air entering the compressor section is compressed and delivered into the combustion section where it is mixed with fuel and ignited to generate a high-speed exhaust gas flow. The high-speed exhaust gas flow expands through the turbine section to drive the compressor and the fan section. The compressor section typically includes low and high pressure compressors, and the turbine section includes low and high pressure turbines.

Lubricant is provided throughout the engine and is circulated through various compartments and structures. Engine lubricant and oil is typically inspected during periodic maintenance for indications of component degradation or failure. Debris in the form of metal particles or other contaminants found in the lubricant can be traced back to a particular component to trigger further inspection. Such periodic monitoring is useful but may not be sufficiently timely to detect potential issues before less costly correction efforts can be utilized. A debris detection system is sometime utilized to detect potential issues based on information obtained from a particle sensor. Debris detection systems includes sensors that are susceptible to providing different readings dependent on different engine operating conditions or environmental conditions. Such variations in sensor data can limit effectiveness. Additionally, errors or failures generated by the detection system can result in false or unreliable information.

Turbine engine manufacturers continue to seek further improvements to engine performance including improvements to thermal, transfer and propulsive efficiencies.

WO 2015/122224 discloses a wind turbine having a light sensor for detecting debris within a lubricant pipe.

EP 2530367 discloses a wind turbine having a sensor that obtains data indicative of debris within a lubricant stream.

EP 2014877 discloses a method for monitoring a gas turbine engine comprising monitoring lubrication oil to detect debris in the oil in order to determine whether the engine is operating within predetermined limits.

### SUMMARY

Viewed from a first aspect, there is provided a method of detecting debris within a lubricant stream of a gas turbine engine, the method comprising: generating data indicative of debris and sensor system functionality within a lubricant stream with a sensor system during operation of the gas turbine engine; communicating the data to a controller; calculating features from the data that are indicative of a debris within the lubricant stream; compiling the calculated features over time during operation of the gas turbine engine; classifying the compiled features; and analysing the compiled features when the gas turbine engine is no longer operating, wherein analysing includes analysis of the compiled features for indications of debris within the lubricant stream indicative of a mechanical anomaly, and/or analysis of the compiled features for indications of an anomaly in operation of the sensor system.

In an embodiment of this aspect, classifying the compiled features includes determining an occurrence rate for the features, which may include comparing the determined occurrence rate with an expected range, and identifying an engine operational characteristic responsive to the comparison with the expected range.

In another embodiment the features include a plurality of features of the lubricant stream that are detected and identified concurrently.

In another embodiment the features include at least one of a time-rate of generation and an overall accumulated number of occurrences.

In another embodiment a time-rate of generation that corresponds with a desired operation of a turbine engine is compared to the determined time-rate of generation to identify an engine operational characteristic.

Viewed from another aspect, there is provided an oil debris monitoring system configured for use in a gas turbine engine, the oil debris monitoring system comprising: a sensor assembly for mounting proximate to a lubricant conduit for generating a signal indicative of particles within a lubricant stream flowing through the lubricant conduit; a feature generator configured to receive the signal from the sensor assembly and generate a data stream including features representing characteristics of the lubricant stream during operation of the gas turbine engine; a feature accumulator configured to compile the features during an operational period of the turbine engine and storing the compiled features in a memory device; and a fault isolator configured to classify the compiled features after the operational period is concluded and generate an output identifying anomalies in the features.

In an embodiment of this aspect, the fault isolator identifies anomalies indicative of a fault in the sensor assembly.

In another embodiment the fault isolator classifies the features according to an occurrence rate for the features, wherein the features may include a plurality of features of the lubricant stream that are detected and identified concurrently and/or the features may include at least one of a time-rate of generation and an overall accumulated number of occurrences.

In another embodiment the feature generator and feature accumulator are part of a controller of the turbine engine.

In another embodiment classifying the compiled features includes determining an occurrence rate for the features, preferably including comparing a determined occurrence rate with an expected range, and identifying an engine operational characteristic responsive to the comparison with the expected range.

In another embodiment the features include a plurality of features of the lubricant stream that are detected and identified concurrently.

In another embodiment, the features include at least one of a time-rate of generation and an overall accumulated number of occurrences.

In another embodiment a time-rate of generation that corresponds with a desired operation of the turbine engine is compared to the determined time-rate of generation to identify an operational characteristic.

Although the different examples have the specific components shown in the illustrations, embodiments of this disclosure are not limited to those particular combinations. It is possible to use some of the components or features from one of the examples in combination with features or components from another one of the examples.

These and other features disclosed herein can be best understood from the following specification and drawings, the following of which is a brief description.

### BRIEF DESCRIPTION OF THE DRAWINGS

Figure 1 schematically shows an embodiment of a gas turbine engine.
Figure 2 schematically shows an example sensor assembly for detecting particles within a lubricant stream.
Figure 3 is a flow diagram schematically showing an example oil debris monitoring system embodiment.

### DETAILED DESCRIPTION

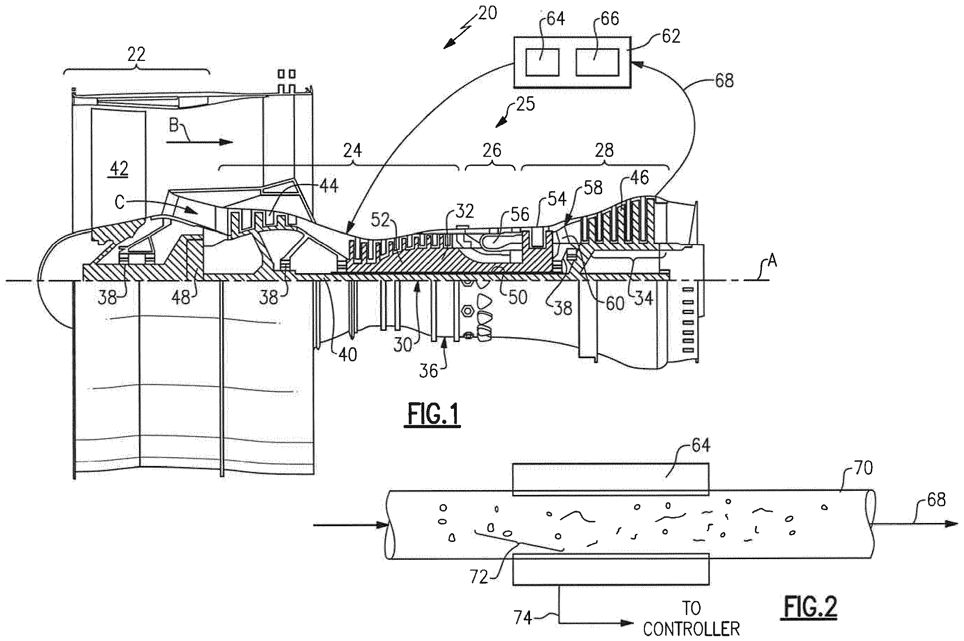

Figure 1 schematically illustrates an example gas turbine engine 20 that includes a fan section 22, a compressor section 24, a combustor section 26 and a turbine section 28. Alternative engines might include an augmenter section (not shown) among other systems or features. The fan section 22 drives air along a bypass flow path B while the compressor section 24 draws air in along a core flow path C where air is compressed and communicated to a combustor section 26. In the combustor section 26, air is mixed with fuel and ignited to generate a high energy exhaust gas stream that expands through the turbine section 28 where energy is extracted and utilized to drive the fan section 22 and the compressor section 24.

Although the disclosed non-limiting embodiment depicts a two-spool turbofan gas turbine engine, it should be understood that the concepts described herein are not limited to use with two-spool turbofans as the teachings may be applied to other types of turbine engines; for example a turbine engine including a three-spool architecture in which three spools concentrically rotate about a common axis and where a low spool enables a low pressure turbine to drive a fan via a gearbox, an intermediate spool that enables an intermediate pressure turbine to drive a first compressor of the compressor section, and a high spool that enables a high pressure turbine to drive a high pressure compressor of the compressor section.

The example engine 20 generally includes a low speed spool 30 and a high speed spool 32 mounted for rotation about an engine central longitudinal axis A relative to an engine static structure 36 via several bearing systems 38. It should be understood that various bearing systems 38 at various locations may alternatively or additionally be provided.

The low speed spool 30 generally includes an inner shaft 40 that connects a fan 42 and a low pressure (or first) compressor section 44 to a low pressure (or first) turbine section 46. The inner shaft 40 drives the fan 42 through a speed change device, such as a geared architecture 48, to drive the fan 42 at a lower speed than the low speed spool 30. The high-speed spool 32 includes an outer shaft 50 that interconnects a high pressure (or second) compressor section 52 and a high pressure (or second) turbine section 54. The inner shaft 40 and the outer shaft 50 are concentric and rotate via the bearing systems 38 about the engine central longitudinal axis A.

A combustor 56 is arranged between the high pressure compressor 52 and the high pressure turbine 54. In one example, the high pressure turbine 54 includes at least two stages to provide a double stage high pressure turbine 54. In another example, the high pressure turbine 54 includes only a single stage. As used herein, a "high pressure" compressor or turbine experiences a higher pressure than a corresponding "low pressure" compressor or turbine.

The example low pressure turbine 46 has a pressure ratio that is greater than about 5. The pressure ratio of the example low pressure turbine 46 is measured prior to an inlet of the low pressure turbine 46 as related to the pressure measured at the outlet of the low pressure turbine 46 prior to an exhaust nozzle.

A mid-turbine frame 58 of the engine static structure 36 is arranged generally between the high pressure turbine 54 and the low pressure turbine 46. The mid-turbine frame 58 further supports bearing systems 38 in the turbine section 28 as well as setting airflow entering the low pressure turbine 46.

Airflow through the core airflow path C is compressed by the low pressure compressor 44 then by the high pressure compressor 52 mixed with fuel and ignited in the combustor 56 to produce high speed exhaust gases that are then expanded through the high pressure turbine 54 and low pressure turbine 46. The mid-turbine frame 58 includes vanes 60, which are in the core airflow path and function as an inlet guide vane for the low pressure turbine 46. Utilizing the vane 60 of the mid-turbine frame 58 as the inlet guide vane for low pressure turbine 46 decreases the length of the low pressure turbine 46 without increasing the axial length of the mid-turbine frame 58. Reducing or eliminating the number of vanes in the low pressure turbine 46 shortens the axial length of the turbine section 28. Thus, the compactness of the gas turbine engine 20 is increased and a higher power density may be achieved.

The disclosed gas turbine engine 20 in one example is a high-bypass geared aircraft engine. In a further example, the gas turbine engine 20 includes a bypass ratio greater than about six (6), with an example embodiment being greater than about ten (10). The example geared architecture 48 is an epicyclical gear train, such as a planetary gear system, star gear system or other known gear system, with a gear reduction ratio of greater than about 2.3.

In one disclosed embodiment, the gas turbine engine 20 includes a bypass ratio greater than about ten (10:1) and the fan diameter is significantly larger than an outer diameter of the low pressure compressor 44. It should be understood, however, that the above parameters are only exemplary of one embodiment of a gas turbine engine including a geared architecture and that the present disclosure is applicable to other gas turbine engines.

A significant amount of thrust is provided by the bypass flow B due to the high bypass ratio. The fan section 22 of the engine 20 is designed for a particular flight condition -- typically cruise at about 0.8 Mach and about 35,000 feet. The flight condition of 0.8 Mach and 35,000 ft., with the engine at its best fuel consumption - also known as "bucket cruise Thrust Specific Fuel Consumption ('TSFC')" - is the industry standard parameter of pound-mass (lbm) of fuel per hour being burned divided by pound-force (lbf) of thrust the engine produces at that minimum point.

"Low fan pressure ratio" is the pressure ratio across the fan blade alone, without a Fan Exit Guide Vane ("FEGV") system. The low fan pressure ratio as disclosed herein according to one non-limiting embodiment is less than about 1.50. In another non-limiting embodiment the low fan pressure ratio is less than about 1.45.

"Low corrected fan tip speed" is the actual fan tip speed in ft/sec divided by an industry standard temperature correction of [(Tram °R)/ (518.7°R)]^{0.5}. The "Low corrected fan tip speed", as disclosed herein according to one non-limiting embodiment, is less than about 1150 ft/second.

The example gas turbine engine includes the fan 42 that comprises in one non-limiting embodiment less than about 26 fan blades. In another non-limiting embodiment, the fan section 22 includes less than about 20 fan blades. Moreover, in one disclosed embodiment the low pressure turbine 46 includes no more than about 6 turbine rotors schematically indicated at 34. In another non-limiting example embodiment the low pressure turbine 46 includes about 3 turbine rotors. A ratio between the number of fan blades 42 and the number of low pressure turbine rotors is between about 3.3 and about 8.6. The example low pressure turbine 46 provides the driving power to rotate the fan section 22 and therefore the relationship between the number of turbine rotors 34 in the low pressure turbine 46 and the number of blades 42 in the fan section 22 disclose an example gas turbine engine 20 with increased power transfer efficiency.

Gas turbine engines designs are seeking to increase not only overall efficiency but also to increase engine monitoring capabilities to detect potential problems to enable corrective action prior to engine function being adversely effected. The example gas turbine engine includes an oil debris monitoring (ODM) system 62 that detects contaminants within a lubricant stream 68. It should be appreciated that throughout this application the terms "oil" and "lubricant" are used interchangeable.

The ODM system 62 includes at least one sensor assembly 64 disposed within a lubricant passage that provides information to a controller 66. The controller 66 may be an overall engine controller or an independent controller. The ODM system 62 detects debris and provides information indicative of debris to the controller 66. Depending on the contaminants detected in the lubricant stream, the controller may prompt a further inspection or other maintenance activity.

Referring to Figure 2 with continued reference to Figure 1, the example ODM system 62 includes the sensor assembly 64 that surrounds or is part of a conduit 70 through which the lubricant stream 68 flows. The lubricant stream 68 may include contaminants schematically illustrated at 72. The contaminants 72 come from various locations in the engine that are supplied oil and can indicate potential mechanical issues that require attention. The contaminants 72 can be identified by their material characteristics, shape, size or other features sensed by the sensor 64. The sensor 64 provides an output signal 74 that is sent to the controller 66. The controller 66 generates data from the signal 74 that is compiled for latter analysis to determine operational health of the turbine engine 20.

In this example, the sensor 64 generates an electrical signal that varies due to contaminants 72 within the lubricant stream 68. The signal 74 varies based on the electrical characteristics of the lubricant stream 68. The electrical characteristics of the lubricant stream 68 vary due to different types of contaminants 72. The contaminants 72 may be ferrous or non-ferrous and may vary in size, shape and quantity for a given time.

The example sensor 64 uses electrical characteristics of the lubricant stream 74 to generate the signal 74, however, other sensors that generate signals that change depending on the presence of contaminants 72 within the lubricant stream are also within the contemplation of this disclosure and may be utilized in the disclosed oil debris monitoring system 62.

In previous oil debris monitoring systems, the information provided by the sensor 64 was processed immediately to identify specific contaminant characteristics and thereby identify specific types of contaminants within the stream. A discrete and separate amount of data would be analyzed individually to determine a specific characteristic that identified with a known contaminant material. Such prior systems detect specific types of particles in the lubricant stream 68 based on a preset definitions for contaminant types. These preset definitions were not always able to account for variations and characteristics of contamination that were detected during different engine operating conditions. As appreciated, each gas turbine engine may include different types of contaminants based on specific operating conditions and environment. A fixed or standard set of conditions is difficult to develop that efficiently covers all types of engines in every type of environment.

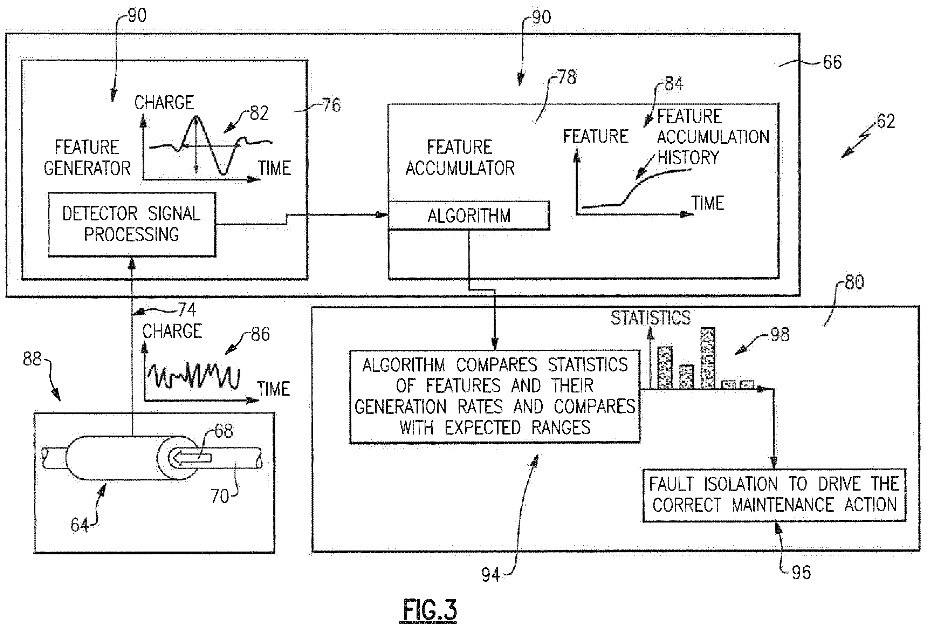

Referring to Figure 3 with continued reference to Figure 1, the example ODM system 62 processes and detects particles and contaminants within a lubricant stream in a manner that accommodates variation in engines and operational environments. Moreover the disclosed ODM system 62 generates multiple calculated statistical features from the sensor signal 74 to enable subsequent analysis, classification and anomaly detection.

The example system 62 includes at least one sensor assembly 64 that generates a signal 74 that is sent to a controller 66. The example controller 66 is mounted within an aircraft including the turbine engine 20. It is within the contemplation of this disclosure that the controller 66 may be a separate dedicated controller or part of the engine controller.

The controller 66 includes a feature generator 76 that receives the signal 74 and generates a feature, illustrated schematically and indicated at 82, from the signal that are indicative of some characteristic of the lubricant flow 68. The features generated are statistical properties that are calculated and determined from the sensor signal 76. The features calculated are selected depending on the available data and the desired characteristic of interest. The features calculated can include frequency content of the signals, projection of the signal onto a wavelet or other basic functions including local peak maximums and minimums, local root mean square, variance, skewness, kurtosis, and other higher statistical moments as are known and understood in the art.

The controller 66 further includes a feature accumulator 78 that compiles the calculated features 82 as is schematically shown at 84. The features 82 are compiled over time for off-line processing. The statistics of each feature 82 may be compiled according to a time-rate of generation and/or for an overall accumulated number of occurrences. Some of the features 82 are associated with normal healthy engine operation, while other features 82 may be indicative of a possible mechanical problem.

A fault isolator 80 is illustrated schematically and receives the compiled features 84 from the feature accumulator 78. The fault isolator 78 classifies the compiled features 84 off-line. Off-line is utilized to indicate a period of time when data is not being generated by the sensor 64 and is most often associated with the completion of a flight cycle where the engine 20 is shut down. The fault isolator 80 may be part of the controller 66 or maybe a separate system utilized for the analysis of the accumulated features 84. The fault isolator 80 classifies the accumulated features 84 for analysis and to drive required maintenance activities. Anomalies in the features 84 can be indicative of mechanical issues with the engine 20 or faults in the sensor assembly 64.

Operation of the example ODM system 62 includes the first step of detecting particles and contaminants within a lubricant stream with the sensor assembly 64 schematically indicated at 88. The sensor assembly 64 generates a signal indicative of debris 72 in the lubricant stream 68. In the disclosed example, the sensor assembly 64 generates an electrical signal indicative of a charge that varies based on electrical characteristics of the lubricant stream as schematically shown at 86. The data signal 74 from the sensor 64 is a continuous stream of data that continues uninterrupted during operation.

The data signal 74 is received by the feature generator 76 that performs the next step indicated at 90 of generating statistical features that represent a condition and content of the lubricant stream 68 over time. The feature generator 76 processes the signal 74 continually to generate a plurality of different statistical features (one shown schematically at 82) that correspond to different characteristics of interest of the lubricant stream 68. The features that are generated from the signal 74 are in the form of statistically calculated properties that can include any known statistical parameter and/or feature. The calculated features can include frequency content of the signals, projection of the signal onto a wavelet or other basic functions including local peak maximums and minimums, local root mean square, variance, skewness, kurtosis, and other higher statistical moments as are known and understood in the art. The feature generator calculates continuously, concurrently and independently multiple features that are subsequently categorization off-line for detection of anomalies.

The calculated features 82 are combined in the feature accumulator 78 over time during operation as is schematically indicated at 92. The features 82 are compiled and /or combined in different ways to reflect engine operation and to group relevant and similar information. The compiling and/or combining of features may be performed according to time-rate of generation for each feature, or a according to an accumulated number of occurrences for latter off-line processing.

The compiled features 84 are next analyzed by the fault isolator 80 as is indicated at 94. Analysis may be performed during operation of the engine, during non-operating conditions, and/or after expiration of a set time. Non-operating conditions can include engine idling and periods during operation after a period in which no issues are detected. The fault isolator 80 is where a comparison of the accumulated features 84 are finally compared to expected ranges indicative of mechanical failure or of sensor malfunction. Because the accumulated features 84 are analyzed off-line, they can reflect an entire operating cycle of the engine and are therefore better suited to identify specific anomalies in particle and contaminants within the lubricant stream 68. Moreover, the off-line analysis also provides a means of identifying a baseline of engine operation that corresponds with heathy engine operating conditions.

The off-line analysis includes classification of the compiled features as is schematically indicated at 98. In this example, classification includes determining an occurrence rate for the calculated features 82. The determined occurrence rate is reviewed to determine how it compares to an expected range. The occurrence rate corresponds with an identified engine operational characteristic and a corresponding directive for a maintenance activity.

Furthermore, noise or interference for a specific engine can be identified and filtered. In previous systems, noise and interference specific to a unique operating environment or engine would cloud analysis and reduce effectiveness. The example system provides continuous and complete features compilations that enable baseline determination for each specific engine and operating environment. Thus, enabling detection of both mechanical anomalies indicative of actual issues with the engine and anomalies that are indicative of sensor faults.

Once the compiled features are classified and analyzed for anomalies, the ODM system 62 provides an output that drives a desired corrective maintenance activity indicated at 96. The activity 96 may range from a further inspection to replacement of an engine component or attention to the sensor assembly 64.

Accordingly, the example system concurrently calculates all features and pertinent statistics and delays the classification and anomaly detection until the system is offline and no longer actively monitoring for particles in real time. By performing the anomaly detection and classification after the system is offline, the systems availability to detect particles is increased. Moreover, because the system is continually generating data it is possible to determine and identify failures for intermittent signal or sensor failures of the system.

Although an example embodiment has been disclosed, a worker of ordinary skill in this art would recognize that certain modifications would come within the scope of this invention. For that reason, the following claims should be studied to determine the scope and content of this invention.

## Claims

1. A method of detecting debris within a lubricant stream of a gas turbine engine (20), the method comprising:
generating data indicative of debris and sensor system functionality within a lubricant stream (68) with a sensor system (64) during operation of the gas turbine engine;
communicating the data to a controller (66);
calculating features from the data that are indicative of a debris within the lubricant stream;
compiling the calculated features over time during operation of the gas turbine engine;
classifying the compiled features; and
analysing the compiled features when the gas turbine engine is no longer operating, wherein analysing includes analysis of the compiled features for indications of debris (72) within the lubricant stream (68) indicative of a mechanical anomaly, and/or analysis of the compiled features for indications of an anomaly in operation of the sensor system.

2. The method as recited in claim 1, wherein classifying the compiled features includes determining an occurrence rate for the features.

3. The method as recited in claim 2, including comparing the determined occurrence rate with an expected range, and identifying an engine operational characteristic responsive to the comparison with the expected range.

4. The method as recited in claim 1, 2 or 3, wherein the features comprise a plurality of features of the lubricant stream (68) that are detected and identified concurrently.

5. The method as recited in any of claims 1 to 4, wherein the features comprise at least one of a time-rate of generation and an overall accumulated number of occurrences, preferably wherein a time-rate of generation that corresponds with a desired operation of a turbine engine is compared to the determined time-rate of generation to identify an engine operational characteristic.

6. The method as recited in claim 1, wherein the sensor system measures electrical characteristics of the lubricant stream (68) and generates an electrical signal that varies due to different types of debris within the lubricant stream.

7. An oil debris monitoring system (62) configured for use in a gas turbine engine (20), the oil debris monitoring system comprising:
a sensor assembly (64) for mounting proximate to a lubricant conduit (70) for generating a signal (74) indicative of particles (72) within a lubricant stream (68) flowing through the lubricant conduit;
a feature generator (76) configured to receive the signal from the sensor assembly and generate a data stream including features representing characteristics of the lubricant stream during operation of the gas turbine engine;
a feature accumulator (78) configured to compile the features during an operational period of the turbine engine and storing the compiled features in a memory device; and
a fault isolator (80) configured to classify the compiled features after the operational period is concluded and generate an output identifying anomalies in the features.

8. The system (62) as recited in claim 7, wherein the fault isolator (80) is configured to identify anomalies indicative of a fault in the sensor assembly (64).

9. The system (62) as recited in claim 7 or 8, wherein the fault isolator (80) is configured to classify the features according to an occurrence rate for the features.

10. The system (62) as recited in claim 9, wherein the features comprise a plurality of features of the lubricant stream (68) that are detected and identified concurrently, and/or wherein the features comprise at least one of a time-rate of generation and an overall accumulated number of occurrences.

11. The system (62) as recited in any of claims 7 to 10, wherein the feature generator (76) and feature accumulator (78) are part of a controller (66) of the turbine engine (20).

12. The system (62) as recited in any of claims 7 to 11, wherein classifying the compiled features includes determining an occurrence rate for the features, preferably including comparing a determined occurrence rate with an expected range, and identifying an engine operational characteristic responsive to the comparison with the expected range.

13. The system (62) as recited in any of claims 7 to 12, wherein the features comprise a plurality of features of the lubricant stream (68) that are detected and identified concurrently.

14. The system (62) as recited in any of claims 7 to 13, wherein the features comprise at least one of a time-rate of generation and an overall accumulated number of occurrences, preferably wherein a time-rate of generation that corresponds with a desired operation of the turbine engine is compared to the determined time-rate of generation to identify an operational characteristic.

15. The system (62) as recited in claim 7, wherein the sensor assembly (64) is configured to measure electrical characteristics of the lubricant stream (68) and to generate an electrical signal that varies due to different types of debris within the lubricant stream.

## Patentansprüche

1. Verfahren zum Erkennen von Ablagerungen in einem Schmiermittelstrom eines Gasturbinentriebwerks (20), wobei das Verfahren Folgendes umfasst:
Erzeugen von Daten, die auf Ablagerungen und eine Sensorsystemfunktionalität in einem Schmiermittelstrom (68) hinweisen, mit einem Sensorsystem (64) während eines Betriebs des Gasturbinentriebwerks;
Kommunizieren der Daten an eine Steuerung (66);
Berechnen von Merkmalen aus den Daten, die auf eine Ablagerung in dem Schmiermittelstrom hinweisen;
Sammeln der berechneten Merkmale im Laufe der Zeit während eines Betriebs des Gasturbinentriebwerks;
Klassifizieren der gesammelten Merkmale; und
Analysieren der gesammelten Merkmale, wenn das Gasturbinentriebwerk nicht mehr in Betrieb ist, wobei Analysieren eine Analyse der gesammelten Merkmale in Bezug auf Hinweise von Ablagerungen (72) in dem Schmiermittelstrom (68), die auf eine mechanische Anomalie hinweisen, und/oder eine Analyse der gesammelten Merkmale in Bezug auf Hinweise auf eine Anomalie im Betrieb des Sensorsystems beinhaltet.

2. Verfahren nach Anspruch 1, wobei Klassifizieren der gesammelten Merkmale Bestimmen einer Auftrittsrate der Merkmale beinhaltet.

3. Verfahren nach Anspruch 2, das Vergleichen der bestimmten Auftrittsrate mit einem erwarteten Bereich und Identifizieren einer Triebwerksbetriebseigenschaft als Reaktion auf den Vergleich mit dem erwarteten Bereich beinhaltet.

4. Verfahren nach einem der Ansprüche 1, 2 oder 3, wobei die Merkmale eine Vielzahl von Merkmalen des Schmiermittelstroms (68) umfassen, die gleichzeitig erkannt und identifiziert werden.

5. Verfahren nach einem der Ansprüche 1 bis 4, wobei die Merkmale mindestens eines von einer Zeitrate einer Erzeugung und einer gesamten akkumulierten Anzahl von Auftritten umfassen, vorzugsweise wobei eine Zeitrate einer Erzeugung, die einem gewünschten Betrieb eines Turbinentriebwerks entspricht, verglichen wird mit der bestimmten Zeitrate einer Erzeugung, um eine Triebwerksbetriebseigenschaft zu identifizieren.

6. Verfahren nach Anspruch 1, wobei das Sensorsystem elektrische Eigenschaften des Schmiermittelstroms (68) misst und ein elektrisches Signal erzeugt, das aufgrund von verschiedenen Arten von Ablagerungen in dem Schmiermittelstrom variiert.

7. Ölablagerungsüberwachungssystem (62), das zur Verwendung in einem Gasturbinentriebwerk (20) konfiguriert ist, wobei das Ölablagerungsüberwachungssystem Folgendes umfasst:
eine Sensoranordnung (64) zum Befestigen in der Nähe einer Schmiermittelleitung (70) zum Erzeugen eines Signals (74), das auf Partikel (72) in einem Schmiermittelstrom (68) hinweist, der durch die Schmiermittelleitung fließt;
einen Merkmalerzeuger (76), der dazu konfiguriert ist, das Signal von der Sensoranordnung zu empfangen und einen Datenstrom zu erzeugen, der Merkmale beinhaltet, die Eigenschaften des Schmiermittelstroms während eines Betriebs des Gasturbinentriebwerks repräsentieren;
einen Merkmalakkumulator (78), der dazu konfiguriert ist, die Merkmale während eines Betriebszeitraums des Gasturbinentriebwerks zu sammeln und die gesammelten Merkmale in einer Speichervorrichtung zu speichern; und
einen Fehlerisolator (80), der dazu konfiguriert ist, die gesammelten Merkmale zu klassifizieren, nachdem der Betriebszeitraum beendet ist, und um eine Ausgabe zu erzeugen, die Anomalien in den Merkmalen identifiziert.

8. System (62) nach Anspruch 7, wobei der Fehlerisolator (80) dazu konfiguriert ist, Anomalien zu identifizieren, die auf einen Fehler in der Sensoranordnung (64) hinweisen.

9. System (62) nach Anspruch 7 oder 8, wobei der Fehlerisolator (80) dazu konfiguriert ist, die Merkmale gemäß einer Auftrittsrate der Merkmale zu klassifizieren.

10. System (62) nach Anspruch 9, wobei die Merkmale eine Vielzahl von Merkmalen des Schmiermittelstroms (68) umfassen, die gleichzeitig erkannt und identifiziert werden, und/oder wobei die Merkmale mindestens eines von einer Zeitrate einer Erzeugung und einer gesamten akkumulierten Anzahl von Auftritten umfassen.

11. System (62) nach einem der Ansprüche 7 bis 10, wobei der Merkmalerzeuger (76) und Merkmalakkumulator (78) Teil einer Steuerung (66) des Turbinentriebwerks (20) sind.

12. System (62) nach einem der Ansprüche 7 bis 11, wobei Klassifizieren der gesammelten Merkmale Bestimmen einer Auftrittsrate der Merkmale beinhaltet, vorzugsweise beinhaltend Vergleichen einer bestimmten Auftrittsrate mit einem erwarteten Bereich und Identifizieren einer Triebwerksbetriebseigenschaft, als Reaktion auf den Vergleich mit dem erwarteten Bereich.

13. System (62) nach einem der Ansprüche 7 bis 12, wobei die Merkmale eine Vielzahl von Merkmalen des Schmiermittelstroms (68) umfassen, die gleichzeitig erkannt und identifiziert werden.

14. System (62) nach einem der Ansprüche 7 bis 13, wobei die Merkmale mindestens eines von einer Zeitrate einer Erzeugung und einer gesamten akkumulierten Anzahl von Auftritten umfassen, vorzugsweise wobei eine Zeitrate einer Erzeugung, die einem gewünschten Betrieb des Turbinentriebwerks entspricht, mit der bestimmten Zeitrate einer Erzeugung verglichen wird, um eine Betriebseigenschaft zu identifizieren.

15. System (62) nach Anspruch 7, wobei die Sensoranordnung (64) dazu konfiguriert ist, elektrische Eigenschaften des Schmiermittelstroms (68) zu messen und ein elektrisches Signal zu erzeugen, das aufgrund von verschiedenen Arten von Ablagerungen in dem Schmiermittelstrom variiert.

## Revendications

1. Procédé de détection de débris à l'intérieur d'un flux de graissage d'un moteur à turbine à gaz (20), le procédé comprenant :
la génération de données indiquant des débris et une fonctionnalité de système de capteur à l'intérieur d'un flux de graissage (68) avec un système de capteur (64) pendant le fonctionnement du moteur à turbine à gaz ;
la communication des données à un dispositif de commande (66) ; le calcul de fonctions à partir des données qui indiquent des débris à l'intérieur du flux de graissage ;
la compilation des fonctions calculées au fil du temps pendant le fonctionnement du moteur à turbine à gaz ;
la classification des fonctions compilées ; et
l'analyse des fonctions compilées lorsque le moteur à turbine à gaz ne fonctionne plus, dans lequel l'analyse comporte l'analyse des fonctions compilées pour des indications de débris (72) à l'intérieur du flux de graissage (68) indiquant une anomalie mécanique, et/ou l'analyse des fonctions compilées pour des indications d'une anomalie dans le fonctionnement du système de capteur.

2. Procédé selon la revendication 1, dans lequel la classification des fonctions compilées comporte la détermination d'un taux d'occurrence pour les fonctions.

3. Procédé selon la revendication 2, comportant la comparaison du taux d'occurrence déterminé avec une plage attendue, et l'identification d'une caractéristique de fonctionnement de moteur en réponse à la comparaison avec la plage attendue.

4. Procédé selon la revendication 1, 2 ou 3, dans lequel les fonctions comprennent une pluralité de fonctions du flux de graissage (68) qui sont détectées et identifiées simultanément.

5. Procédé selon l'une quelconque des revendications 1 à 4, dans lequel les fonctions comprennent au moins l'un d'un taux temporel de génération et d'un nombre d'occurrences cumulé total, de préférence dans lequel un taux temporel de génération qui correspond à un fonctionnement souhaité d'un moteur à turbine est comparé avec le taux temporel de génération déterminé pour identifier une caractéristique de fonctionnement de moteur.

6. Procédé selon la revendication 1, dans lequel le système de capteur mesure des caractéristiques électriques du flux de graissage (68) et génère un signal électrique qui varie en raison de différents types de débris à l'intérieur du flux de graissage.

7. Système de supervision de débris d'huile (62) configuré pour être utilisé dans un moteur à turbine à gaz (20), le système de supervision de débris d'huile comprenant :
un ensemble capteur (64) destiné à être monté à proximité d'une ligne de graissage (70) pour générer un signal (74) indiquant des particules (72) à l'intérieur d'un flux de graissage (68) s'écoulant à travers la ligne de graissage ;
un générateur de fonctions (76) configuré pour recevoir le signal depuis l'ensemble capteur et générer un flux de données comportant des fonctions représentant des caractéristiques du flux de graissage pendant le fonctionnement du moteur à turbine à gaz ;
un accumulateur de fonctions (78) configuré pour compiler les fonctions pendant une période de fonctionnement du moteur à turbine et stocker les fonctions compilées dans un dispositif de mémoire ; et
un isolateur de défaut (80) configuré pour classifier les fonctions compilées après la fin de la période de fonctionnement et générer une sortie identifiant des anomalies dans les fonctions.

8. Système (62) selon la revendication 7, dans lequel l'isolateur de défaut (80) est configuré pour identifier des anomalies indiquant un défaut dans l'ensemble capteur (64).

9. Système (62) selon la revendication 7 ou 8, dans lequel l'isolateur de défaut (80) est configuré pour classifier les fonctions en fonction d'un taux d'occurrence pour les fonctions.

10. Système (62) selon la revendication 9, dans lequel les fonctions comprennent une pluralité de fonctions du flux de graissage (68) qui sont détectées et identifiées simultanément, et/ou dans lequel les fonctions comprennent au moins l'un d'un taux temporel de génération et d'un nombre d'occurrences cumulé total.

11. Système (62) selon l'une quelconque des revendications 7 à 10, dans lequel le générateur de fonctions (76) et l'accumulateur de fonctions (78) font partie d'un dispositif de commande (66) du moteur à turbine (20).

12. Système (62) selon l'une quelconque des revendications 7 à 11, dans lequel la classification des fonctions compilées comporte la détermination d'un taux d'occurrence pour les fonctions, comportant de préférence la comparaison d'un taux d'occurrence déterminé avec une plage attendue, et l'identification d'une caractéristique de fonctionnement de moteur en réponse à la comparaison avec la plage attendue.

13. Système (62) selon l'une quelconque des revendications 7 à 12, dans lequel les fonctions comprennent une pluralité de fonctions du flux de graissage (68) qui sont détectées et identifiées simultanément.

14. Système (62) selon l'une quelconque des revendications 7 à 13, dans lequel les fonctions comprennent au moins l'un d'un taux temporel de génération et d'un nombre d'occurrences cumulé total, de préférence dans lequel un taux temporel de génération qui correspond à un fonctionnement souhaité du moteur à turbine est comparé avec le taux temporel de génération déterminé pour identifier une caractéristique de fonctionnement.

15. Système (62) selon la revendication 7, dans lequel l'ensemble capteur (64) est configuré pour mesurer des caractéristiques électriques du flux de graissage (68) et pour générer un signal électrique qui varie en raison de différents types de débris à l'intérieur du flux de graissage.
